# EUROPEAN PATENT SPECIFICATION

(11) **EP 1 388 529 B1**
(45) Date of publication and mention of the grant of the patent: **16.07.2014**
(21) Application number: 03254691.3
(22) Date of filing: 28.07.2003
(51) Int. Cl.: A01N 29/04, A01N 29/08

(54) **Cyclopropane precursors of ethylene response inhibitors in plants**
Cyclopropane-Vorläufer zur Inhibierung der Ethylen-Antwort von Pflanzen
Précurseurs cyclopropaniques des inhibiteurs d´une réponse à l'éthylène dans les plantes

(30) Priority: 06.08.2002 US 401308 P
(43) Date of publication of application: 11.02.2004
(73) Proprietor: ROHM AND HAAS COMPANY, Philadelphia, Pennsylvania 19106-2399 (US)
(72) Inventor: Jacobson, Richard Martin, Chalfont Pennsylvania 18914 (US); Kelly, Martha Jean, Collegeville Pennsylvania 19426 (US); James, William Nixon, Jr., Hatfield Pennsylvania 19440 (US)
(74) Representative: Kent, Venetia Katherine

(56) References cited:
- EP-A- 0 629 626
- WO-A-02/068609
- US-A- 5 518 988
- US-A- 5 611 210
- US-A- 6 017 849
- DATABASE CROSSFIRE BEILSTEIN [Online] Beilstein Institut zur Förderung der Chemischen Wissenschaften, Frankfurt am Main, DE; Database accession no. 302174 XP002263828 & DEMJANOW: CHEM.ZENTRALBL., vol. 102, no. I, 1931, page 930
- DATABASE CROSSFIRE BEILSTEIN [Online] Beilstein Institut zur Förderung der Chemischen Wissenschaften, Frankfurt am Main, DE; Database accession no. 302173 XP002263829 & DEMJANOW: CHEM.BER., vol. 56, 1923, page 2201
- DATABASE CROSSFIRE BEILSTEIN [Online] Beilstein Institut zur Förderung der Chemischen Wissenschaften, Frankfurt am Main, DE; Database accession no. 3710333 XP002263830 & S.HARTMANN: CHEM. PHYS. LIPIDS, vol. 71, no. 1, 1994, pages 99-108,
- DATABASE CROSSFIRE BEILSTEIN [Online] Beilstein Institut zur Förderung der Chemischen Wissenschaften, Frankfurt am Main, DE; Database accession no. 3457258 XP002263831 & H. NEUNHOEFFER: LIEBIGS ANN. CHEM., vol. 4, 1993, pages 367-374,
- DATABASE CROSSFIRE BEILSTEIN [Online] Beilstein Institut zur Förderung der Chemischen Wissenschaften, Frankfurt am Main, DE; Database accession no. 3503375;3509478 XP002263832 & S. MARK BAIRD: J. CHEM. SOC. PERKIN TRANS. 1, vol. 14, 1993, pages 1547-1548, XP000902822
- S. W. TOBEY: "Tetracyclopropene, tetrabromopropene and some fluorinated cyclopropenes and cyclopropanes." J. AM. CHEM. SOC., vol. 88, no. 11, 1966, pages 2481-2488, XP002263825
- K. B. WIBERG: "Cyclopropene" J. AM. CHEM. SOC., vol. 82, 1960, pages 6375-6380, XP002263826
- M.F.SEMMELHACK: "Synthesis and thermal rearrangements of spiro[2.3]hexadiene and spiro[2.3]hex-4-ene derivatives" J.AM.CHEM.SOC., vol. 94, no. 25, 1972, pages 8838-8847, XP002263827
- DATABASE CROSSFIRE BEILSTEIN [Online] Beilstein Institut zur Förderung der Chemischen Wissenschaften, Frankfurt am Main, DE; Database accession no. 2446517 XP002263833 & R. J. AL-DULAYYMI: TETRAHEDRON, vol. 46, no. 16, 1990, pages 5703-5714,
- DATABASE CROSSFIRE BEILSTEIN [Online] Beilstein Institut zur Förderung der Chemischen Wissenschaften, Frankfurt am Main, DE; Database accession no. 1058452 XP002263834 & E. VOGEL: ANGEW. CHEM., vol. 87, 1975, pages 591-592,
- P.A. WADE ET AL.: "Polynitro-substituted strained-ring compounds" J.AM.CHEM.SOC., vol. 113, 1991, pages 8807-8811, XP002274492
- K. B. WIBERG ET AL.: "1,2-brigded cyclopropenes" J. AM.CHEM.SOC., vol. 113, 1991, pages 7969-7979, XP002274493
- J.B. LAMBERT ET AL.: "Stereomutationin the Seyfert reaction" J.AM.CHEM.SOC., vol. 107, 1985, pages 5443-5447, XP002274494
- K.L.WILLIAMSON ET AL.: "F19 coupling constants and chemical shifts in trifluorocyclopropanes" J.AM.CHEM.SOC., vol. 89, 1967, pages 6183-6186, XP002274495
- E.C. SISLEY ET AL.: "Inhibitors of ethylene responses in plants at the receptor level" PHYSIOLOGIA PLANTARUM, vol. 100, 1997, pages 577-582, XP000218586
- J.M. BIRCHHALL ET AL.: J.CHEM.SOC. PERKIN TRANS.1, vol. 14, 1973, pages 1773-1779, XP000902822
- S. MARK BAIRD ET AL.: J.CHEM.SOC.PERKIN TRANS.1, vol. 3, 1993, pages 321-326, XP000902822
- H.A.STEFANI ET AL.: SYNTH.COMM., vol. 28, no. 9, 1998, pages 1667-1678, XP000902822
- M.COONEY ET AL.: AUST.J.CHEM., vol. 49, no. 4, 1996, pages 533-538, XP000902823
- A. DULAYYMI ET AL.: "2-Vinyl-1,1,2-trihalocyclopropanes" TETRAHEDRON, vol. 52, no. 33, 1996, pages 10955-10968, XP002274500
- A. DULAYYMI ET AL.: "1,2,2-Tribromocyclopropanecarboxylic acid and derivatives" TETRAHEDRON, vol. 52, no. 10, 1996, pages 3409-3424, XP002274501
- A.DULAYYMI ET AL.: "Pyridazines by addition of diazoalkanes to 1-bromo- and 1,2-dibromocyclopropenes" TETRAHEDRON, vol. 54, no. 42, 1998, pages 12897-12906, XP002274502
- J.L. SCHLATTER: "The preparation of cyclopropene" J.AM.CHEM.SOC., vol. 63, 1941, pages 1733-1737, XP002274581
- A.DULAYYMI ET AL.: "A flexible route to 1-bromo-2-alkylcyclopropenes" J.CHEM.SOC.PERKIN TRANS.1, 1994, pages 1547-1548, XP000902823
- P.MÜLLER ET AL.: "Synthesis of cycloproparenes via aromatization of 7-oxanorbornenes with low-valent titanium" HELV.CHIMICA ACTA, vol. 72, 1989, pages 1608-1617, XP000902823
- DATABASE CAPLUS [Online] CHEMICAL ABSTRACTS SERVICE, COLUMBUS, OHIO, US SIMS C.; WEGE D.: 'Cyclopropa-fusion to seven-membered unsaturated rings.' Database accession no. 1995:458169
- DATABASE CAPLUS [Online] CHEMICAL ABSTRACTS SERVICE, COLUMBUS, OHIO, US CHENIER P. ET AL: 'Synthesis and chemistry of some tricyclic cyclopropenes.' Database accession no. 1993:408389
- DATABASE CAPLUS [Online] CHEMICAL ABSTRACTS SERVICE, COLUMBUS, OHIO, US BAIRD M.S. ET AL: 'Formation of diiodocyclopropanes from electron-poor alkenes' Database accession no. 1986:497027

## Description

The present invention relates to inhibiting the ethylene response in plants or plant parts. Plant parts include, for example, flowers, leaves, fruits and vegetables and may remain on the parent plant or may be harvested. The ethylene response accelerates the ripening of the plant or, especially, the harvested plant part, such as a fruit or vegetable. Such accelerated ripening makes it necessary to transport such products as quickly as possible, under optimum conditions, to the final consumer before the harvested product is rendered unmarketable by becoming prematurely rotten.

It is well known that plants contain molecular receptor sites for the molecule ethylene. Ethylene affects many plant characteristics, specifically those related to plant growth, development and senescence. For the harvester of plant products, such as fruits and vegetables, ethylene causes most problems in the area of senescence. Specifically, once fruits and vegetables are harvested, ethylene will cause these products to ripen and eventually rot at an accelerated rate. Much work has been done in an effort to either eliminate or mitigate the deleterious effects of ethylene on harvested plant products.

An example of an irreversible ethylene inhibiting agent is disclosed in U.S. Patent 5,100,462. This patent discloses diazocyclopentadiene as the blocking agent. However, this compound exhibits a strong odor and is very unstable. In an effort around these problems, U.S. Patent 5,518,988 discloses the discovery of cyclopropene and derivatives thereof, which are used as effective blocking agents for the ethylene binding site. However, while the compounds of this patent do not suffer from the odor problems of diazocyclopentadiene they are relatively unstable gases. Therefore, the stability of these gases, as well as the explosive potential these gases pose when compressed still present problems.

Since the cyclopropenes of the '988 patent have proven to be very effective ethylene inhibitors, it remains very desirable to find a viable means to resolve their instability problem. One approach that was taken is disclosed in U.S. Patent 6,017,849. This patent shows that it is possible to encapsulate the cyclopropene molecule into a cyclodextrin molecule as a carrier. This approach allows for the safe storage and transport of the cyclopropene/cyclodextrin complex, in general providing a shelf life of more than one year.

Although the foregoing encapsulation technique provides a substantially more stable ethylene inhibiting agent, problems still remain. For instance, the double bond in the cyclopropene molecule is very reactive and makes the molecule susceptible to degradation under a variety of storage and handling conditions.

Therefore, what is needed is an ethylene inhibitor that is storage stable over a long period of time, is not susceptible to self-degradation and eliminates the significant risk of explosion associated with the handling of cyclopropenes. The present invention solves these problems by utilizing certain precursors of the cyclopropene class of ethylene inhibitor molecules. These precursors have increased storage stability. In practice, the precursors are converted to their corresponding cyclopropene molecule when treatment of the target plant parts is desired.

The present invention relates to stabilizing unstable cyclopropene molecules by converting them to their more stable cyclopropane analogs. The double bond is eliminated by binding moieties to each carbon atom component of the double bond. In the formulae of the disclosure of this invention, these moieties are designated as W1 and W2. These stabilizing moieties are selected from F, Cl, Br, I, alkoxy, acyloxy, alkoxycarbonyloxy, aminocarbonyloxy, alkylaminocarbonyloxy, dialkylaminocarbonyloxy, alkylsulfonyloxy and arylsulfonyloxy groups; with the proviso that at least one of W1 and W2 is a Br or I.

The present invention, in its various aspects, is as set out in the accompanying claims.

According to one aspect, the present invention comprises a method to inhibit the ethylene response in plants with a cyclopropene compound comprising providing cyclopropene derivatives of structures I, II, III and IV. These compounds are represented as follows:

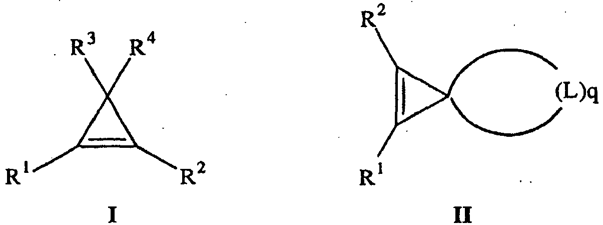

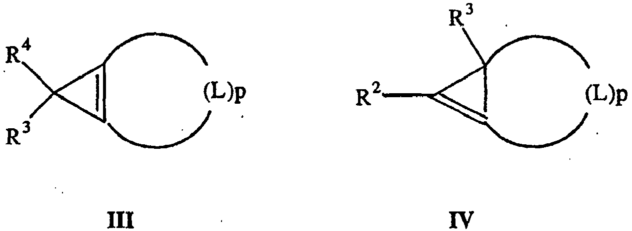

Structures I, II, III and IV represent cyclopropene derivative compounds which are effective ethylene antagonists. These compounds are derived from their respective cyclopropane precursor molecules V, VI, VII and VIII:

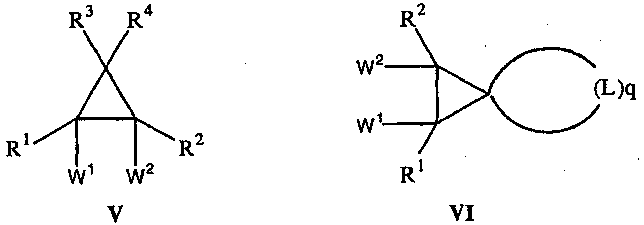

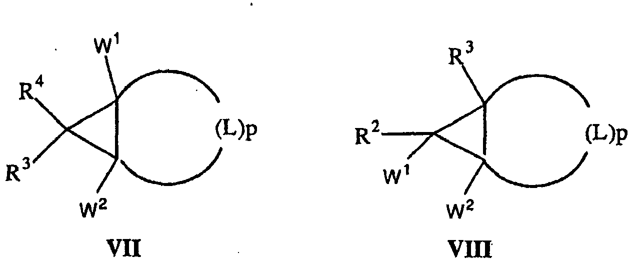

Compounds of structures V, VI, VII and VIII are reacted with a reducing agent or a nucleophile to obtain the respective gaseous compounds of structures I, II, III, and IV. Compounds I, II, III and IV are thus released into the target enclosed atmosphere to treat the plants or plant parts to inhibit the ethylene response.

According to a second aspect, the present invention comprises a method of using any one of a compound of structures V, VI, VII and VIII wherein:
a) each R¹, R², R³, and R⁴ is independently a group of the formula:

   -(L)ₙ-Z

   wherein:
   i) p is an integer from 3 to 10;
      q is an integer from 4 to 11;
      n is an integer from 0 to 12;
   ii) each L is independently selected from a member of the group D, E, or J wherein:
      D is of the formula: 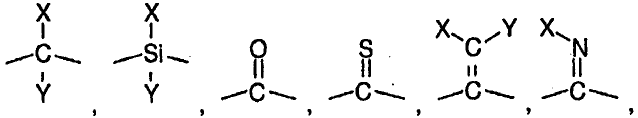 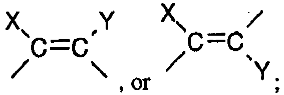
      E is of the formula: 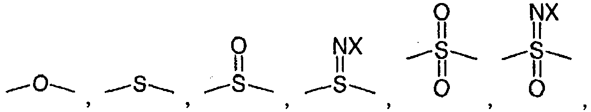 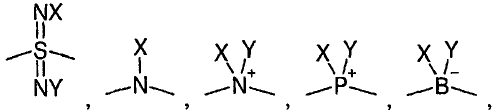 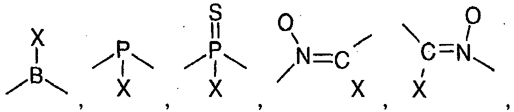 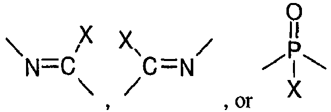 and
      J is of the formula: 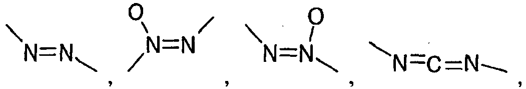 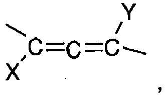 or

         -C≡C-

         wherein:
         A) each X and Y is independently a group of the formula:

            -(L)ₘ-Z;

            and
         B) m is an integer from 0 to 8; and
         C) no more than two E groups are adjacent to each other and no J groups are adjacent to each other;
   iii) each Z is independently selected from:
      A) hydrogen, halo, cyano, nitro, nitroso, azido, chlorate, bromate, iodate, isocyanato, isocyanido, isothiocyanato, pentafluorothio, or
      B) a group G, wherein G is an unsubstituted or substituted; unsaturated, partially saturated, or saturated; monocyclic, bicyclic, tricyclic, or fused; carbocyclic or heterocyclic ring system wherein;
         1) when the ring system contains a 3 or 4 membered heterocyclic ring, the heterocyclic ring contains 1 heteroatom;
         2) when the ring system contains a 5, or more, membered heterocyclic ring or a polycyclic heterocyclic ring, the heterocyclic or polycyclic heterocyclic ring contains from 1 to 4 heteroatoms;
         3) each heteroatom is independently selected from N, O, and S;
         4) the number of substituents is from 0 to 5 and each substituent is independently selected from X;
b) W¹ and W² are selected from F, Cl, Br, I, alkoxy, acyloxy, alkoxycarbonyloxy, aminocarbonyloxy, alkylaminocarbonyloxy, dialkylaminocarbonyloxy, alkylsulfonyloxy, and arylsulfonyloxy;
c) at least one of W¹ and W² is a Br or I; and
d) the total number of non-hydrogen atoms in each compound is 50 or less; as a plant ethylene response antagonist by contacting the plant with said compound.

For the purposes of this invention, in the structural representations of the various L groups, each open bond indicates a bond to another L group, a Z group, or the cyclopropene moiety. For example, the structural representation 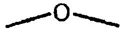 indicates an oxygen atom with bonds to two other atoms; it does not represent a dimethyl ether moiety.

Typical R¹, R², R³, and R⁴ groups include, for example: alkenyl, alkyl, alkynyl, acetylaminoalkenyl, acetylaminoalkyl, acetylaminoalkynyl, alkenoxy, alkoxy, alkynoxy, alkoxyalkoxyalkyl, alkoxyalkenyl, alkoxyalkyl, alkoxyalkynyl, alkoxycarbonylalkenyl, alkoxycarbonylalkyl, alkoxycarbonylalkynyl, alkylcarbonyl, alkylcarbonyloxyalkyl, alkyl(alkoxyimino)alkyl, carboxyalkenyl, carboxyalkyl, carboxyalkynyl, dialkylamino, haloalkoxyalkenyl, haloalkoxyalkyl, haloalkoxyalkynyl, haloalkenyl, haloalkyl, haloalkynyl, hydroxyalkenyl, hydroxyalkyl, hydroxyalkynyl, trialkylsilylalkenyl, trialkylsilylalkyl, trialkylsilylalkynyl, dialkylphosphonato, dialkylphosphato, dialkylthiophosphato, dialkylaminoalkyl, alkylsulfonylalkyl, alkylthioalkenyl, alkylthioalkyl, alkylthioalkynyl, dialkylaminosulfonyl, haloalkylthioalkenyl, haloalkylthioalkyl, haloalkylthioalkynyl, alkoxycarbonyloxy; cycloalkenyl, cycloalkyl, cycloalkynyl, acetylaminocycloalkenyl, acetylaminocycloalkyl, acetylaminocycloalkynyl, cycloalkenoxy, cycloalkoxy, cycloalkynoxy, alkoxyalkoxycycloalkyl, alkoxycycloalkenyl, alkoxycycloalkyl, alkoxycycloalkynyl, alkoxycarbonylcycloalkenyl, alkoxycarbonylcycloalkyl, alkoxycarbonylcycloalkynyl, cycloalkylcarbonyl, alkylcarbonyloxycycloalkyl, carboxycycloalkenyl, carboxycycloalkyl, carboxycycloalkynyl, dicycloalkylamino, halocycloalkoxycycloalkenyl, halocycloalkoxycycloalkyl, halocycloalkoxycycloalkynyl, halocycloalkenyl, halocycloalkyl, halocycloalkynyl, hydroxycycloalkenyl, hydroxycycloalkyl, hydroxycycloalkynyl, trialkylsilylcycloalkenyl, trialkylsilylcycloalkyl, trialkylsilylcycloalkynyl, dialkylaminocycloalkyl, alkylsulfonylcycloalkyl, cycloalkylcarbonyloxyalkyl, cycloalkylsulfonylalkyl, alkylthiocycloalkenyl, alkylthiocycloalkyl, alkylthiocycloalkynyl, dicycloalkylaminosulfonyl, haloalkylthiocycloalkenyl, haloalkylthiocycloalkyl, haloalkylthiocycloalkynyl; aryl, alkenylaryl, alkylaryl, alkynylaryl, acetylaminoaryl, aryloxy, alkoxyalkoxyaryl, alkoxyaryl, alkoxycarbonylaryl, arylcarbonyl, alkylcarbonyloxyaryl, carboxyaryl, diarylamino, haloalkoxyaryl, haloaryl, hydroxyaryl, trialkylsilylaryl, dialkylaminoaryl, alkylsulfonylaryl, arylsulfonylalkyl, alkylthioaryl, arylthioalkyl, diarylaminosulfonyl, haloalkylthioaryl; heteroaryl, alkenylheteroaryl, alkylheteroaryl, alkynylheteroaryl, acetylaminoheteroaryl, heteroaryloxy, alkoxyalkoxyheteroaryl, alkoxyheteroaryl, alkoxycarbonylheteroaryl, heteroarylcarbonyl, alkylcarbonyloxyheteroaryl, carboxyheteroaryl, diheteroarylamino, haloalkoxyheteroaryl, haloheteroaryl, hydroxyheteroaryl, trialkylsilylheteroaryl, dialkylaminoheteroaryl, alkylsulfonylheteroaryl, heteroarylsulfonylalkyl, alkylthioheteroaryl, heteroarylthioalkyl, diheteroarylaminosulfonyl, haloalkylthioheteroaryl; heterocyclyl, alkenylheteroycycyl, alkylheteroycycyl, alkynylheteroycycyl, acetylaminoheterocyclyl, heterocyclyloxy, alkoxyalkoxyheterocyclo, alkoxyheterocyclyl, alkoxycarbonylheterocyclyl, heterocyclylcarbonyl, alkylcarbonyloxyheterocyclyl, carboxyheterocyclyl, diheterocyclylamino, haloalkoxyheterocyclyl, haloheterocyclyl, hydroxyheterocyclyl, trialkylsilylheterocyclyl, dialkylaminoheterocyclyl, alkylsulfonylheterocyclyl, alkylthioheterocyclyl, heterocyclylthioalkyl, diheterocyclylaminosulfonyl, haloalkyllthioheterocyclyl; hydrogen, fluoro, chloro, bromo, iodo, cyano, nitro, nitroso, azido, chlorato, bromato, iodato, isocyanato, isocyanido, isothiocyanato, pentafluorothio; acetoxy, carboethoxy, cyanato, nitrato, nitrito, perchlorato, allenyl; butylmercapto, diethylphosphonato, dimethylphenylsilyl, isoquinolyl, mercapto, naphthyl, phenoxy, phenyl, piperidino, pyridyl, quinolyl, triethylsilyl, trimethylsilyl.

Typical G groups include, for example: saturated or unsaturated cycloalkyl, bicyclic, tricyclic, polycyclic, saturated or unsaturated heterocyclic, unsubstituted or substituted phenyl, naphthyl, or heteroaryl ring systems such as, for example, cyclopropyl, cyclobutyl, cyclopent-3-en-1-yl, 3-methoxycyclohexan-1-yl, phenyl, 4-chlorophenyl, 4-fluorophenyl, 4-bromophenyl, 3-nitrophenyl, 2-methoxyphenyl, 2-methylphenyl, 3-methyphenyl, 4-methylphenyl, 4-ethylphenyl, 2-methyl-3-methoxyphenyl, 2,4-dibromophenyl, 3,5-difluorophenyl, 3,5-dimethylphenyl, 2,4,6-trichlorophenyl, 4-methoxyphenyl, naphthyl, 2-chloronaphthyl, 2,4-dimethoxyphenyl, 4-(trifluoromethyl)phenyl, 2- iodo-4-methylphenyl, pyridin-2-yl, pyridin-3-yl, pyridin-4-yl, pyrazinyl, pyrimidin-2-yl, pyrimidin-4-yl, pyrimidin-5-yl, pyridazinyl, triazol-1-yl, imidazol-1-yl, thiophen-2-yl, thiophen-3-yl, furan-2-yl, furan-3-yl, pyrrolyl, oxazolyl, isoxazolyl, thiazolyl, isothiazolyl, oxadiazolyl, thiadiazolyl, quinolyl, isoquinolyl, tetrahydrofuryl, pyrrolidinyl, piperidinyl, tetrahydropyranyl, morpholinyl, piperazinyl, dioxolanyl, dioxanyl, indolinyl and 5-methyl-6-chromanyl, adamantyl, norbornyl, and their substituted analogs such as, for example: 3-butyl-pyridin-2-yl, 4-bromo-pyridin-2-yl, 5-carboethoxy-pyridin-2-yl, 6-methoxyethoxy-pyridin-2-yl.

Preferably, two of R¹, R², R³, and R⁴ are hydrogen. More preferably, R¹ and R² are hydrogen or R³ and R⁴ are hydrogen. Even more preferably, R², R³, and R⁴ are hydrogen or R¹, R², and R³ are hydrogen. Most preferably, R², R³, and R⁴ are hydrogen.

Preferably, n is from 0 to 8. Most preferably, n is from 1 to 7. Preferably, m is 0 to 4. Most preferably, m is from 0 to 2.

Preferably, D is -CXY-, -SiXY-, -CO-, or -CS-. More preferably D is -CXY-. Preferably, E is -O-, -S-, -NX-, or -SO₂-. Preferably, X and Y are independently H, halo, OH, SH, -C(O)(C₁-C₄)alkyl -, -C(O)O(C₁-C₄)alkyl -, -O-(C₁-C₄)alkyl, -S-(C₁-C₄)alkyl, or substituted or unsubstituted (C₁-C₄)alkyl. Preferably, Z is H, halo, or G. More preferably, Z is H or G.

Preferably, each G is independently a substituted or unsubstituted; five, six, or seven membered; aryl, heteroaryl, heterocyclic, or cycloalkyl ring. More preferably, each G is independently a substituted or unsubstituted phenyl, pyridyl, cyclohexyl, cyclopentyl, cycloheptyl, pyrolyl, furyl, thiophenyl, triazolyl, pyrazolyl, 1,3-dioxolanyl, or morpholinyl. Even more preferably, G is unsubstituted or substituted phenyl, cyclopentyl, cycloheptyl, or cyclohexyl. Most preferably, G is cyclopentyl, cycloheptyl, cyclohexyl, phenyl, or substituted phenyl wherein the substituents are independently selected from 1 to 3 of methyl, methoxy, and halo.

In one aspect, the method of the present invention comprises converting the precursor compounds of structures V, VI, VII and VIII into the corresponding ethylene antagonistic compounds of structures I, II, III, and IV, respectively. This is achieved by reacting the compound of structures V, VI, VII or VIII with a reducing or a nucleophilic agent. The moieties identified as W1 and W2 on structures V, VI, VII and VIII are often referred to as "leaving groups". These groups will remain on the core molecule until cleaved off by reaction with, as in this instance, a reducing or nucleophilic agent. Once the reducing or nucleophilic agent cleaves off the leaving group, the molecule of structures V, VI, VII and VIII converts to the molecule of structures I, II, III and IV, respectively.

Reducing agents may be classified as metals, organometallic reagents and low valent metal ions. Suitable examples of metals are zinc, magnesium, iron, copper, samarium and aluminum. Examples of organometallic reagents are methyllithium and n-butyllithium. Low valent metal ions include Cr(II), Ti(II), Cu(I) and Fe(II). The most preferred reducing agent is metallic zinc.

Nucleophilic agents include mercaptans, selenides, phosphines, phosphites, Na2S, Na2Te, Na2S2O4, diethylphosphite sodium salt, KSCN, NaSeCN, thiourea, diphenyltelurium and NaI. These nucleophiles may also be incorporated into polymeric reagents.

Molecules of structure V are preferred in the practice of this invention. The most preferred molecule is where R1=CH3, R2=H, R3=H, R4=H, W1=I and W2=I. This molecule is identified as 1,2-diiodo-1-methylcyclopropane. In the practice of this invention, this molecule represents a stable precursor to the ethylene antagonist 1-methylcylopropene. The following reaction shows the conversion from the stable 1,2-diiodo-1-methylcyclopropane to the gaseous 1-methylcylopropene upon reaction with zinc.

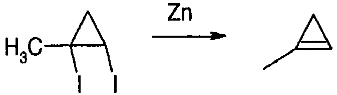

A number of examples were prepared. Different leaving groups are also exemplified. Although 77 examples were actually prepared, it is only necessary to show a few reaction schemes. The number of the example correlates with the same number in the list of structures identified.

### Example 23

### 1,1,2-tribromocyclopropane

Into a 3000 ml three necked round bottomed flask equipped with a mechanical stirrer was added 350 g of bromoform, 575 g of methylene chloride, 130 g of vinyl bromide, 4.5 g of N,N'-dibenzyl-N,N,N',N'-tetramethylethylenediammonium dibromide and 60 g of 45% aqueous potassium hydroxide. After stirring for two days, 500 ml of water was added and the organic layer was separated. An additional 4.5 g of N,N'-dibenzyl-N,N,N',N'-tetramethylethylenediammonium dibromide and 60 g of 45% aqueous potassium hydroxide were added and stirring was resumed overnight. After washing with water, the organic layer was distilled yielding 1,1,2-tribromocyclopropane bp (10 torr) 75 - 80 °C. nmr (CDCl₃) δ 1.72 (t, 1H), 2.76 (t, 1H), 3.58 (t, 1H).

### Example 37

### Preparation of 1-Hexyl-1,2,2-tribromocyclopropene

### a. 2-Bromo-oct-1-ene

A solution of 9.42 ml (0.0728 mol) of 2,3-dibromopropene in 70 ml diethylether was placed under a nitrogen atmosphere by use of a Firestone valve. While cooling in an ice water bath, a solution of 0.091 mol of pentylmagnesium bromide in 70 ml diethyl ether was added slowly via addition funnel. After stirring for 2 hours while warming to room temperature, there was then added via syringe 50 ml of 1 N hydrochloric acid to the reaction cooling in an ice water bath. The resulting mixture was transferred to a separatory funnel and the phases were separated. The organic layer was dried over MgSO₄ and filtered. The solvent was removed from the filtrate *in vacuo* to yield 15.0 g (85.7% of theory) of 81% pure 2-bromo-oct-1-ene as an oil.

### b. 1,1,2-Tribromo-2-hexyl-cyclopropane

To 5.42 g (28.4 mmol) of 2-bromo-oct-1-ene in 7.42 ml (85.1 mmol) of bromoform and 48.8 ml of methylene chloride, were added 1.30 g (2.84 mmol) of N,N'-dibenzyl-N,N,N',N'-tetramethylethylenediammonium dibromide and 12.1 ml (142 mmol) of 45% aqueous potassium hydroxide. The mixture was stirred at room temperature for 5 days. There was then added hexanes and water. This mixture was filtered. The resulting mixture was transferred to a separatory funnel and the phases were separated. The organic layer was dried over MgSO₄ and filtered. The solvent was removed from the filtrate *in vacuo* to yield 5.25 g (51.0% of theoretical) of 1,1,2-tribromo-2-hexyl-cyclopropane as an oil.

### Example 55

### 1,2-diiodo-1-octylcyclopropane

To 20 g of methyl alcohol was added 1.33 g (16.2 mmole) of anhydrous sodium acetate and 3.3 g (13 mmole) of elemental iodine. The mixture was cooled to 5 °C whereon 2.0 g of 1-octylcyclopropene (13 mmole) [prepared from 1,2,2-tribromo-1-octylcyclopropane by the method of Baird, Mark S.; Hussain, Helmi H.; Nethercott, William; J.Chem.Soc.Perkin Trans. 1, 1986, 1845-1854] The reaction was stirred at room temperature for two hours. The reaction was concentrated *in vacuo* and the product was diluted with hexanes and washed with dilute aqueous sodium hydroxide. Re-concentration *in vacuo* and column chromatography over silica gel gave 1.7 g of the desired 1,2-diiodo-1-octylcyclopropane. nmr (CDCl₃) δ 0.88 (m, 4H), 1.3 (m, 10H), 1.5-1.8 (m, 5H), 3.26 (t, 1H).

### Example 56

### 1,2-diiodo-1 -benzylcyclopropane

1-benzylcyclopropene [prepared from 3.65 g (10.0 mmole) of 1,2,2-tribromo-1-benzylcyclopropane by the method of Baird, Mark S.; Hussain, Helmi H.; Nethercott, William; J.Chem.Soc.Perkin Trans. 1, 1986, 1845-1854] was added to a stirred mixture of 0.77 g (9.4 mmole) of anhydrous sodium acetate and 2.60 g of elemental iodine in 30 g of methanol. After stirring overnight, the reaction was concentrated *in vacuo* and the product was diluted with hexanes and washed with dilute aqueous sodium hydroxide. Re-concentration *in vacuo* and column chromatography over silica gel gave 3.0 g of the desired 1,2-diiodo-1-benzylcyclopropane. nmr (CDCl₃) δ 1.18 (t, 1H), 3.1 (abq, 2H), 3.41 (t, 1H), 7.3 (m, 5H).

### Example 60

### 1,2-diiodo-1-methylcyclopropane

To 300 g of methyl alcohol was added 8.2 g (100 mmole) of anhydrous sodium acetate and 53 g (209 mmole) of elemental iodine. The mixture was cooled to 5 °C whereon 19 g of 1-methylcyclopropene [prepared from 3-chloro-2-methyl-propene; see, for example, Hopf, H.; Wachholz, G.; Walsh, R. Chem. Ber., 118, 3579 (1985), and Köster, R et al., Liebigs Annalen Chem., 1219-1235, (1973).] was added. The reaction was stirred at room temperature until the color lightened. The reaction was concentrated *in vacuo* and the product was diluted with hexanes and washed with dilute aqueous sodium hydroxide. Re-concentration *in vacuo* gave 45.7 g of the desired 1,2-diiodo-1-methylcyclopropane. Bp (5 torr) 76 °C. nmr (CDCl₃) δ 0.88 (t, 1H), 1.71 (t, 1H), 1.99 (s, 3H), 3.22 (t, 1H).

### Example 61

### 1,1-dichloro-2-bromocyclopropane

Into a 3000 ml three necked round bottomed flask equipped with a mechanical stirrer was added 500 g of chloroform, 103 g of vinyl bromide, 5.6 g of N,N'-dibenzyl-N,N,N',N'-tetramethylethylenediammonium dibromide and 200 g of 45% aqueous potassium hydroxide. After stirring for two days, 500 ml of water was added and the organic layer was separated. The organic layer was distilled yielding 1,1-dichloro-2-bromocyclopropane bp (760 torr) 140 - 150 °C. nmr (CDCl₃) δ 1.65 (t, 1H), 2.13 (t, 1H), 3.53 (t, 1H).

### Example 76

### 1,2-diiodocyclopropane

Cyclopropane, made from 10 ml of allyl chloride by the method of Binger [J. Org. Chem. 61, 6462-6464 (1996)] was condensed into a flask containing 10.13 g of iodine, 2 g of pyridine and 100 g of 2-propanol at -70 °C. The reaction mixture was slowly warmed to +10 °C over the course of three hours and concentrated *in vacuo.* The resulting mixture was partitioned between diethyl ether and dilute aqueous hydrochloric acid. Washing the ether layer with dilute aqueous sodium hydroxide, saturated aqueous sodium chloride, drying over anhydrous magnesium sulfate, and concentration *in vacuo* yielded 6.0 g of trans-1,2-diiodocyclopropane which was purified by column chromatography over silica gel. nmr (CDCl₃) δ 1.36 (t, 2H), 2.66 (t, 2H).
Structural examples of compounds produced according to the invention.

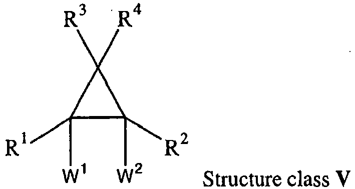

| | R1 | W1 | W2 | R2 | R3 | R4 |
|---|---|---|---|---|---|---|
| 1 | OCTYL | Br | Br | Br | H | H |
| 2 | C6H5 | Br | Br | Br | H | H |
| 3 | CH2CH2C6H5 | Br | Br | Br | H | H |
| 4 | OCTYL | Br | Cl | Cl | H | H |
| 5 | CH2OC6H5 | Br | Br | Br | H | H |
| 6 | C8H17 | Br | Cl | Cl | H | H |
| 7 | CH2OC6H4OMe-4 | Br | Br | Br | H | H |
| 8 | CH2C6H5 | Br | Br | Br | H | H |
| 9 | UNDECYL | Br | Br | Br | H | H |
| 10 | NONYL | Br | Br | Br | H | H |
| 11 | HEPTYL | Br | Br | Br | H | H |
| 12 | DECYL | Br | Br | Br | H | H |
| 13 | (2-CYCLOHEXYLETHYL) | Br | Br | Br | H | H |
| 14 | TRIDECYL | Br | Br | Br | H | H |
| 15 | (3-ETHYLHEPTYL) | Br | Br | Br | H | H |
| 16 | (CYCLOHEPTYLMeTHYL) | Br | Br | Br | H | H |
| 17 | (CYCLOHEXYLMeTHYL) | Br | Br | Br | H | H |
| 18 | CH2C6H4CL-4 | Br | Br | Br | H | H |
| 19 | CH2CH2OH | Br | Br | Br | H | H |
| 20 | CH2OCH2CH2OCH2CH2OMe | Br | Br | Br | H | H |
| 21 | CH2CH2CO2ET | Br | Br | Br | H | H |
| 22 | Br | Br | OET | H | H | H |
| 23 | Br | Br | Br | H | H | H |
| 24 | Br | Br | OBU | Br | H | H |
| 25 | CH2C6H4Me-4 | Br | Br | Br | H | H |
| 26 | CH2CH2CH2C6H5 | Br | Br | Br | H | H |
| 27 | CH2C6H4OMe-2 | Br | Br | Br | H | H |
| 28 | HEPTYL(7-OMe) | Br | Br | Br | H | H |
| 29 | HEPTYL(6-Me) | Br | Br | Br | H | H |
| 30 | CH2CH20PENTYL | Br | Br | Br | H | H |
| 31 | HEPTYL(7-OH) | Br | Br | Br | H | H |
| 32 | CH2CH2CH2CH2C6H5 | Br | Br | Br | H | H |
| 33 | PENTYL | Br | Br | Br | H | H |
| 34 | CH2THIOPHENE-2-YL | Br | Br | Br | H | H |
| 35 | BUTYL | Br | Br | Br | H | H |
| 36 | CH2CH2C6H4CL-4 | Br | Br | Br | H | H |
| 37 | HEXYL | Br | Br | Br | H | H |
| 38 | CH2C6H4Me-3 | Br | Br | Br | H | H |
| 39 | HEPTYL(4,6,6-TRIMETHYL) | Br | Br | Br | H | H |
| 40 | HEXYL(6-CO2H) | Br | Br | Br | H | H |
| 41 | CH2CYCLOPENTYL | Br | Br | Br | H | H |
| 42 | HEXYL(6-OMS) | Br | Br | Br | H | H |
| 43 | Br | Br | Br | H | OCTYL | H |
| 44 | PENTADECYL | Br | Br | Br | H | H |
| 45 | (CH2)4CF3 | Br | Br | Br | H | H |
| 46 | CH2CH2CO2H | Br | Br | Br | H | H |
| 47 | NONYL(4,8-Me2) | Br | Br | Br | H | H |
| 48 | DODECYL | Br | Br | Br | H | H |
| 49 | CH2CH2COMORPHOLINE | Br | Br | Br | H | H |
| 50 | CH2CH(ET)BU | Br | Br | Br | H | H |
| 51 | (CH2)7CN | Br | Br | Br | H | H |
| 52 | (CH2)7NET2 | Br | Br | Br | H | H |
| 53 | TETRADECYL | Br | Br | Br | H | H |
| 54 | TETRADECYL | Br | Br | Br | H | H |
| 55 | OCTYL | I | I | H | H | H |
| 56 | BENZYL | I | I | H | H | H |
| 57 | (3,3-DIMETHYLBUTYL) | Br | Br | Br | H | H |
| 58 | HEXYL | Br | Br | Br | HEXYL | H |
| 59 | METHYL | Br | Br | Br | H | H |
| 60 | METHYL | I | I | H | H | H |
| 61 | Cl | Cl | Br | H | H | H |
| 62 | CH2CH2CH2DIOXANE-2-YL | Br | Br | Br | H | H |
| 63 | CH2CH2CONET2 | Br | Br | Br | H | H |
| 64 | CH2SIET3 | Br | Br | Br | H | H |
| 65 | CH2CH2OCH(Me)OET | Br | Br | Br | H | H |
| 66 | CH2CH2OSO2PH | Br | Br | Br | H | H |
| 67 | (CH2)6SiMe3 | Br | Br | Br | H | H |
| 68 | (CH2)2SiMe3 | Br | Br | Br | H | H |
| 69 | CH2CH2CO2CH2OAC | Br | Br | Br | H | H |
| 70 | C(Me)(Me)C6H5 | Cl | Br | Br | H | H |
| 71 | (CH2)6SiMe2Ph | Br | Br | Br | H | H |
| 72 | CH2Ph | Br | Cl | Cl | H | H |
| 73 | Me | Br | Br | Me | Me | Me |
| 74 | (CH2)40COC6H4Me-4 | Br | Br | Br | H | H |
| 75 | (CH2)40H | Br | Br | Br | H | H |
| 76 | H | I | I | H | H | H |

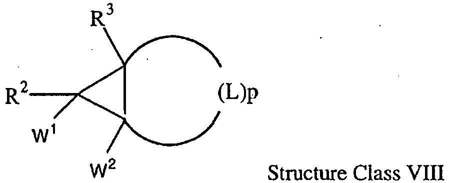

| | | | | | |
|---|---|---|---|---|---|
| | (L)p | W1 | W2 | R2 | R3 |
| 77 | CH2CH2CH2CH2CH2 | Br | Br | Br | H |

### Chemically Induced Release of a cyclopropene

### Control Experiment

Into a 50 ml Florence flask with magnetic stirring was placed 2 ml of tetrahydrofuran and 0.30 g of 1,2-diiodo-1-methylcyclopropane. After stirring for 5 minutes GC analysis of the headspace showed no detectable 1-methylcyclopropene. GC method uses Varian CP-PoraBOND Q column 10 meters long 0.32 mm ID; helium carrier; initial temperature 50 °C; initial time 0 minutes; ramp rate 20 °C/min; final temperature 270 °C; final time 5 minutes; injection volume 0.20 ml. The retention time of an authentic sample of 1-methylcyclopropene was 2.91 minutes. 1 ppm is easily detectable under these conditions.

### 1-methyleyelopropene formation using zinc metal in tetrahydrofuran

Into a 100 ml Florence flask with magnetic stirring was placed 2 ml of tetrahydrofuran and 1.0 g of zinc dust. The zinc was activated with 10 drops of 1,2-dibromoethane. Then 0.34 g of 1,2-diiodo-1-methylcyclopropane was added. After stirring for 20 hours, GC analysis of the headspace showed 4658 ppm 1-methylcyclopropene.

### 1-methylcyclopropene formation using zinc metal in methanol

Into a 100 ml Florence flask with magnetic stirring was placed 2 ml of methanol and 1.0 g of zinc dust. The zinc was activated with 10 drops of 1,2-dibromoethane. Then 0.34 g of 1,2-diiodo-1-methylcyclopropane was added. After stirring for 30 minutes GC analysis of the headspace showed 98390 ppm 1-methylcyclopropene.

### 1-methylcylopropene formation using magnesium metal

Into a 100 ml Florence flask with magnetic stirring was placed 2 ml of tetrahydrofuran and 1.1 g of magnesium turnings. The magnesium was activated with 10 drops of 1,2-dibromoethane. Then 0.35 g of 1,2-diiodo-1-methylcyclopropane was added. After stirring for 3 hours GC analysis of the headspace showed 49993 ppm 1-methylcyclopropene.

### 1-methylcyclopropene formation using triphenylphosphine

Into a 50 ml Florence flask with magnetic stirring was placed 3 g of dimethylformamide and 1.2 g of triphenylphosphine. Then 0.83 g of 1,2-diiodo-1-methylcyclopropane was added. After stirring for 15 minutes at room temperature, GC analysis of the headspace showed 10 ppm 1-methylcyclopropene.

### 1-methylcyclopropene formation using 4-methylbenzenethiol

Into a 100 ml Florence flask with magnetic stirring was placed 2 g of dimethylformamide, 0.70 g of potassium t-butoxide, and 0.84 g of 4-methylbenzenethiol. Then 0.40 g of 1,2-diiodo-1-methylcyclopropane was added. After stirring for 15 minutes at room temperature, GC analysis of the headspace showed 87567 ppm 1-methylcyclopropene.

### 1-methylcyclopropene formation using polymer containing benzenethiol groups

The polymeric reagent was prepared by slurrying 50 ml of Duolite™ GT73 (Rohm and Haas Company) and stirring for two hours with 50 ml of water and 10 g of 45% aqueous potassium hydroxide. The slurry was filtered, washed twice with water, thrice with methanol, air dried, and placed in a vacuum oven overnight. 0.54 g of this polymeric reagent was placed in a 122 ml vial and the beads were wetted with 0.10 g of 1,2-diiodo-1-methylcyclopropane in 0.70 g of methanol. After standing overnight at room temperature, GC analysis of the headspace showed 134 ppm of 1-methylcyclopropene.

## Claims

1. A method of using any one of a compound of a structure selected from the group consisting of: 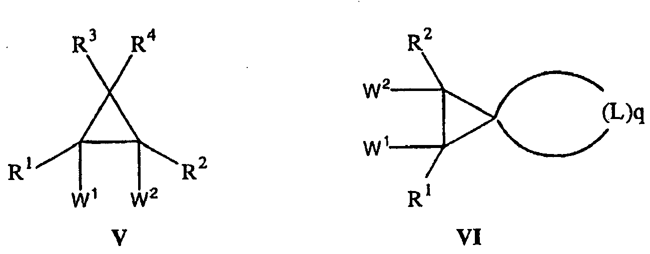 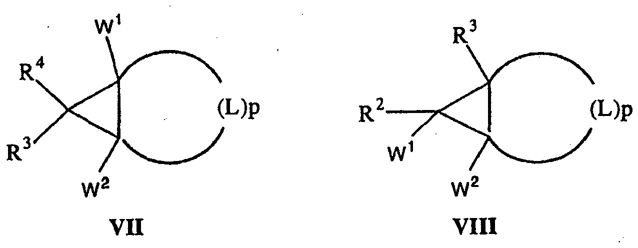 wherein:
a) each R¹, R², R³, and R⁴ is independently a group of the formula:
-(L)ₙ-Z
i) p is an integer from 3 to 10;
q is an integer from 4 to 11;
n is an integer from 0 to 12;
ii) each L is independently selected from a member of the group D, E, or J
D is of the formula: 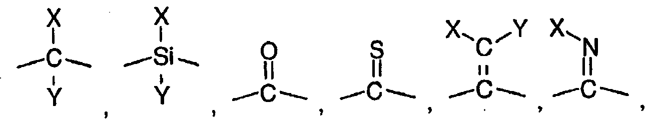 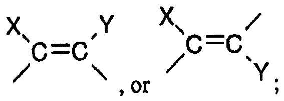 E is of the formula: 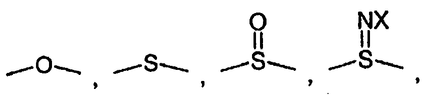 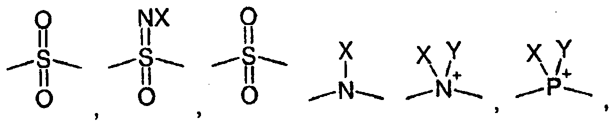 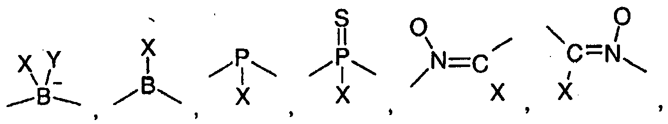 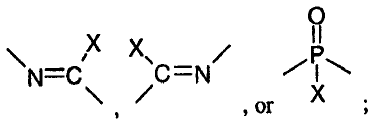 and
J is of the formula: 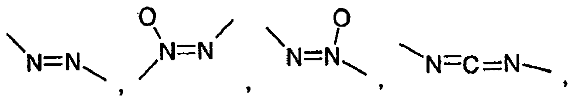 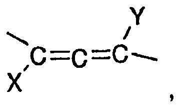
-C≡C-
or
A) each X and Y is independently a group of the formula:
-(L)ₘ-Z;
and
B) m is an integer from 0 to 8; and
C) no more than two E groups are adjacent to each other and no J groups are adjacent to each other;
iii) each Z is independently selected from:
A) hydrogen, halo, cyano, nitro, nitroso, azido, chlorate, bromate, iodate, isocyanato, isocyanido, isothiocyanato, pentafluorothio, or
B) a group G, wherein G is an unsubstituted or substituted; unsaturated, partially saturated, or saturated; monocyclic, bicyclic, tricyclic, or fused; carbocyclic or heterocyclic ring system wherein;
1) when the ring system contains a 3 or 4 membered heterocyclic ring, the heterocyclic ring contains 1 heteroatom;
2) when the ring system contains a 5, or more, membered heterocyclic ring or a polycyclic heterocyclic ring, the heterocyclic or polycyclic heterocyclic ring contains from 1 to 4 heteroatoms;
3) each heteroatom is independently selected from N, O, and S;
4) the number of substituents is from 0 to 5 and each substituent is independently selected from X;
b) W¹ and W² are selected from F, Cl, Br, I, alkoxy, acyloxy, alkoxycarbonyloxy, aminocarbonyloxy, alkylaminocarbonyloxy, dialkylaminocarbonyloxy, alkylsulfonyloxy, and arylsulfonyloxy;
c) provided that at least one of W¹ and W² is I; and
d) the total number of non-hydrogen atoms is 50 or less.
as a plant ethylene response antagonist by contacting the plant with said compound.

2. The method of claim 1 wherein each of W1 and W2 are I.

3. The method of claim 1 wherein the compound is 1,2-diiodo-1-methylcyclopropane.

4. A method to inhibit the ethylene response in plants with a cyclopropene compound comprising the steps of:
A) providing a compound of structure I, II, III or IV 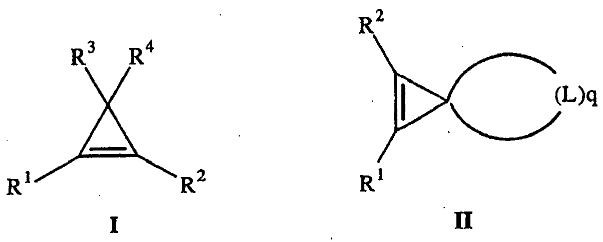 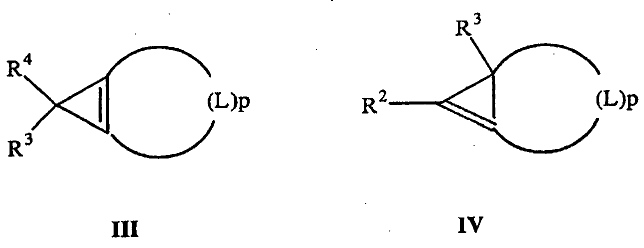 comprising contacting a compound of structure V, VI, VII or VIII of claim 1, with a reducing or nucleophilic agent to convert the compound of structure V, VI, VII or VIII into its respective analogous compound of structure I, II, III or IV, respectively, wherein:
a) each R¹, R², R³, and R⁴ is independently a group of the formula:
-(L)ₙ-Z
i) p is an integer from 3 to 10;
q is an integer from 4 to 11;
n is an integer from 0 to 12;
ii) each L is independently selected from a member of the group D, E, or J :
D is of the formula: 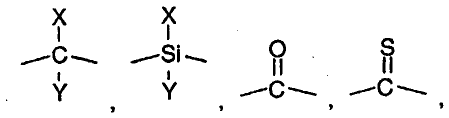 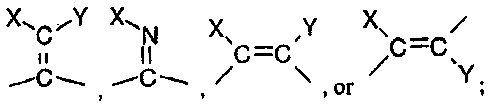
E is of the formula: 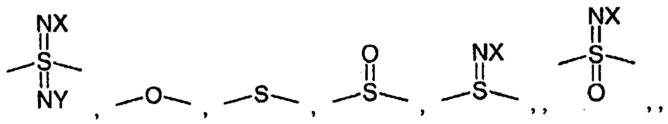 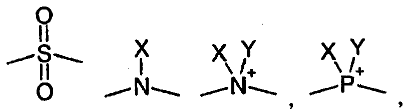 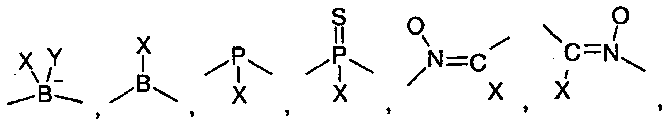 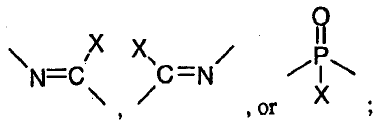 and
J is of the formula: 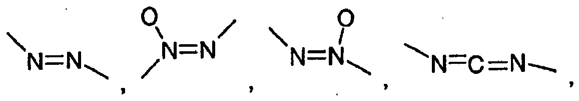 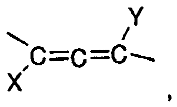 or
-C≡C-
A) each X and Y is independently a group of the formula:
-(L)ₘ-Z;
and
B) m is an integer from 0 to 8; and
C) no more than two E groups are adjacent to each other and no J groups are adjacent to each other;
iii) each Z is independently selected from:
A) hydrogen, halo, cyano, nitro, nitroso, azido, chlorate, bromate, iodate, isocyanato, isocyanido, isothiocyanato, pentafluorothio, or
B) a group G, wherein G is an unsubstituted or substituted; unsaturated, partially saturated, or saturated; monocyclic, bicyclic, tricyclic, or fused; carbocyclic or heterocyclic ring system wherein;
1) when the ring system contains a 3 or 4 membered heterocyclic ring, the heterocyclic ring contains 1 heteroatom;
2) W¹ and W² are selected from F, Cl, Br, I, alkoxy, acyloxy, alkoxycarbonyloxy, aminocarbonyloxy, alkylaminocarbonyloxy, dialkylaminocarbonyloxy, alkylsulfonyloxy, and arylsulfonyloxy;
c) provided that at least one of W¹ and W² is a Br or I; and
d) the total number of non-hydrogen atoms is 50 or less, and
B) contacting the plant with the compound.

5. The method of claim 4 wherein the reducing agent is selected from the group consisting of metals, organometallic reagents and low valent metal ions.

6. The method of claim 4 wherein the nucleophilic agent is selected from the group consisting of mercaptans, selenides, phosphines, phosphites, Na2S, Na2Te, Na2S2O4, diethylphosphite sodium salt, KSCN, NaSeCN, thiourea, diphenyltelurium and NaI.

## Patentansprüche

1. Ein Verfahren zum Verwenden einer beliebigen von einer Verbindung mit einer Struktur, ausgewählt aus der Gruppe, bestehend aus: 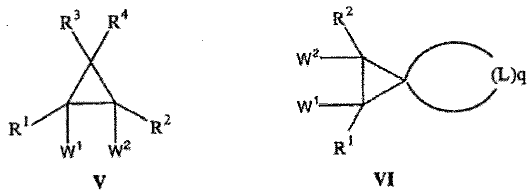 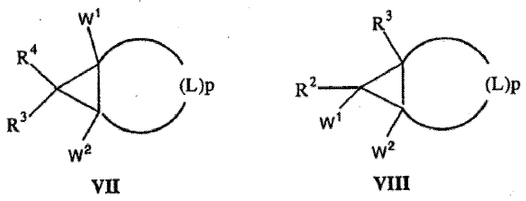 wobei:
a) jeder R¹, R², R³ und R⁴ unabhängig eine Gruppe mit der folgenden Formel ist:
-(L)ₙ-Z
i) p eine ganze Zahl von 3 bis 10 ist;
q eine ganze Zahl von 4 bis 11 ist;
n eine ganze Zahl von 0 bis 12 ist;
ii) jedes L unabhängig ausgewählt ist aus einem Glied der Gruppe D, E oder J,
D die folgende Formel aufweist: 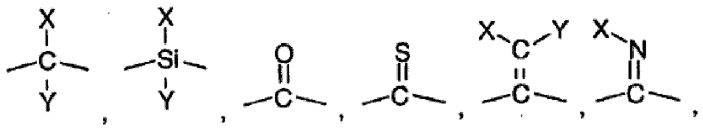 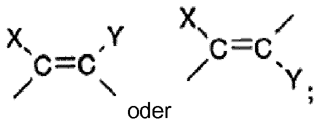 E die folgende Formel aufweist: 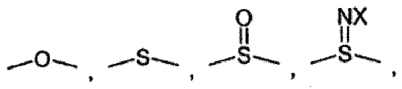 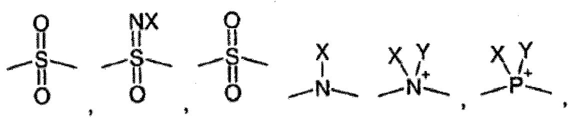 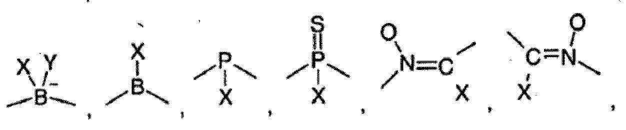 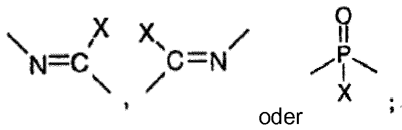 und
J die folgende Formel aufweist: 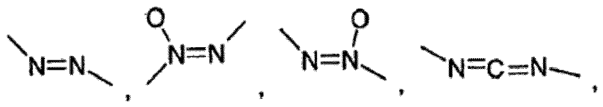 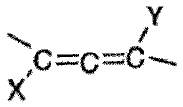
-C≡C-
oder
A) jedes X und Y unabhängig eine Gruppe mit der folgenden Formel ist:
-(L)ₘ-Z;
und
B) m eine ganze Zahl von 0 bis 8 ist; und
C) nicht mehr als zwei E-Gruppen aneinander angrenzen und keine J-Gruppen aneinander angrenzen;
iii) jedes Z unabhängig ausgewählt ist aus:
A) Wasserstoff, Halogen, Cyan, Nitro, Nitroso, Azido, Chlorat, Bromat, Iodat, Isocyanat, Isocyanid, Isothiocyanat, Pentafluorthio oder
B) einer G-Gruppe, wobei G ein unsubstituiertes oder substituiertes; ungesättigtes, teilweise gesättigtes oder gesättigtes; monocyclisches, bicyclisches, tricyclisches oder kondensiertes; carbocyclisches oder heterocyclisches Ringsystem ist, wobei;
1) wenn das Ringsystem einen heterocyclischen Ring mit 3 oder 4 Gliedern enthält, enthält der heterocyclische Ring 1 Heteroatom;
2) wenn das Ringsystem einen heterocyclischen Ring mit 5 oder mehr Gliedern oder einen polycyclischen heterocyclischen Ring enthält, enthält der heterocyclische oder polycyclische heterocyclische Ring von 1 bis 4 Heteroatome;
3) jedes Heteroatom unabhängig ausgewählt ist aus N, O und S;
4) die Zahl an Substituenten von 0 bis 5 ist und jeder Substituent unabhängig ausgewählt ist aus X;
b) W¹ und W² ausgewählt sind aus F, Cl, Br, I, Alkoxy, Acyloxy, Alkoxycarbonyloxy, Aminocarbonyloxy, Alkylaminocarbonyloxy, Dialkylaminocarbonyloxy, Alkylsulfonyloxy und Arylsulfonyloxy;
c) vorausgesetzt, dass mindestens eines von W¹ und W² I ist; und
d) die Gesamtzahl von Nicht-Wasserstoffatomen 50 oder weniger beträgt.
als Pflanzenethylenantwortantagonist durch In-Kontakt-Bringen der Pflanze mit der Verbindung.

2. Verfahren gemäß Anspruch 1, wobei W¹ wie auch W² I ist.

3. Verfahren gemäß Anspruch 1, wobei die Verbindung 1,2-Diiod-1-methycylropropan ist.

4. Ein Verfahren zum Inhibieren der Ethylenantwort bei Pflanzen mit einer Cyclopropenverbindung, beinhaltend die Schritte:
A) Bereitstellen einer Verbindung mit der Struktur I, II, III oder IV 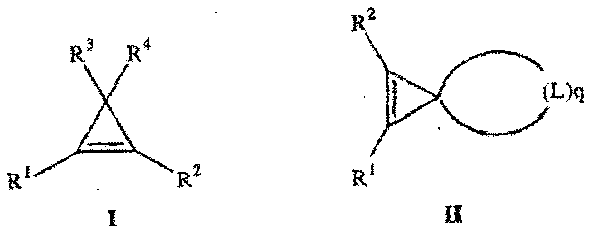 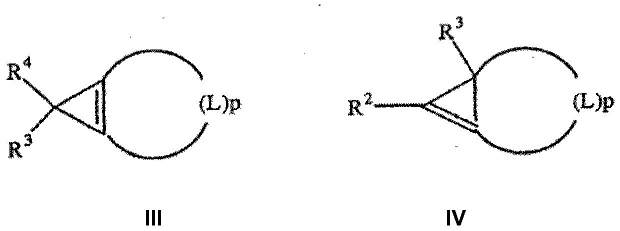 beinhaltend In-Kontakt-Bringen einer Verbindung mit der Struktur V, VI, VII oder VIII gemäß Anspruch 1, mit einem Reduktionsmittel oder nukleophilen Mittel, um die Verbindung mit der Struktur V, VI, VII oder VIII in ihre jeweilige analoge Verbindung mit der Struktur I, II, III bzw. IV umzuwandeln,
wobei:
a) jeder R¹, R², R³ und R⁴ unabhängig eine Gruppe mit der folgenden Formel ist:
-(L)ₙ-Z
i) p eine ganze Zahl von 3 bis 10 ist;
q eine ganze Zahl von 4 bis 11 ist;
n eine ganze Zahl von 0 bis 12 ist;
ii) jedes L unabhängig ausgewählt ist aus einem Glied der Gruppe D, E oder J;
D die folgende Formel aufweist: 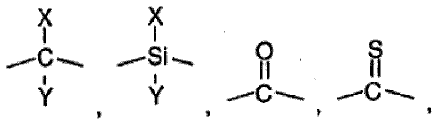 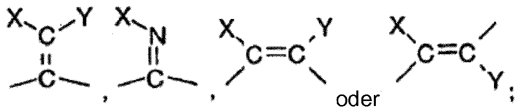 E die folgende Formel aufweist: 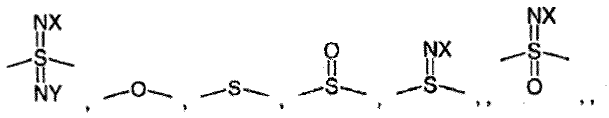 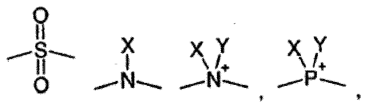 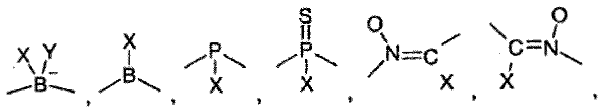 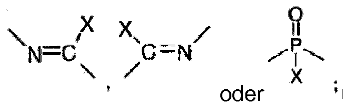 und
J die folgende Formel aufweist: 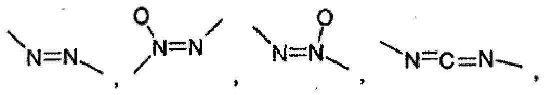 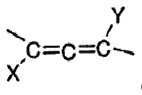
-C≡C-
oder
A) jedes X und Y unabhängig eine Gruppe mit der folgenden Formel ist:
-(L)ₘ-Z;
und
B) m eine ganze Zahl von 0 bis 8 ist; und
C) nicht mehr als zwei E-Gruppen aneinander angrenzen und keine J-Gruppen aneinander angrenzen;
iii) jedes Z unabhängig ausgewählt ist aus:
A) Wasserstoff, Halogen, Cyan, Nitro, Nitroso, Azido, Chlorat, Bromat, Iodat, Isocyanat, Isocyanid, Isothiocyanat, Pentafluorthio oder
B) einer G-Gruppe, wobei G ein unsubstituiertes oder substituiertes; ungesättigtes, teilweise gesättigtes oder gesättigtes; monocyclisches, bicyclisches, tricyclisches oder kondensiertes; carbocyclisches oder heterocyclisches Ringsystem ist, wobei;
1) wenn das Ringsystem einen heterocyclischen Ring mit 3 oder 4 Gliedern enthält, enthält der heterocyclische Ring 1 Heteroatom;
2) W¹ und W² ausgewählt sind aus F, Cl, Br, I, Alkoxy, Acyloxy, Alkoxycarbonyloxy, Aminocarbonyloxy, Alkylaminocarbonyloxy, Dialkylaminocarbonyloxy, Alkylsulfonyloxy und Arylsulfonyloxy;
c) vorausgesetzt, dass mindestens eines von W¹ und W² ein Br oder I ist; und
d) die Gesamtzahl von Nicht-Wasserstoffatomen 50 oder weniger beträgt, und
B) In-Kontakt-Bringen der Pflanze mit der Verbindung.

5. Verfahren gemäß Anspruch 4, wobei das Reduktionsmittel ausgewählt ist aus der Gruppe, bestehend aus Metallen, Organometallreaktanten und Niedervalent-Metallionen.

6. Verfahren gemäß Anspruch 4, wobei das nukleophile Mittel ausgewählt ist aus der Gruppe, bestehend aus Mercaptanen, Seleniden, Phosphinen, Phosphiten, Na2S, Na2Te, Na2S2O4, Diethylphosphit-Natriumsalz, KSCN, NaSeCN, Thioharnstoff, Diphenyltellur und Nal.

## Revendications

1. Une méthode pour utiliser un composé quelconque parmi un composé d'une structure sélectionnée dans le groupe consistant en : 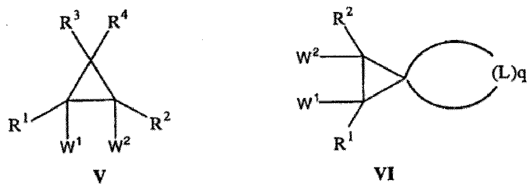 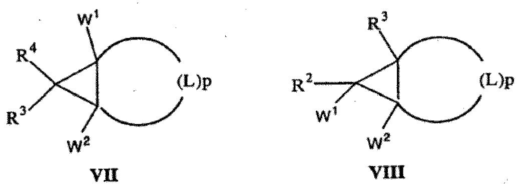 dans lesquelles :
a) chaque R¹, R², R³, et R⁴ est indépendamment un groupe de la formule :
-(L)ₙ-Z
i) p est un nombre entier allant de 3 à 10 ;
q est un nombre entier allant de 4 à 11 ;
n est un nombre entier allant de 0 à 12 ;
ii) chaque L est indépendamment sélectionné parmi un élément du groupe D, E, ou J
D est de la formule : 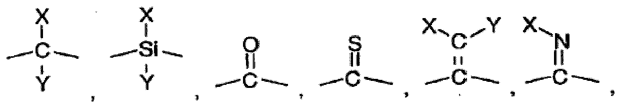 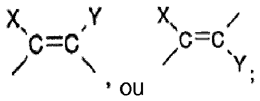 E est de la formule : 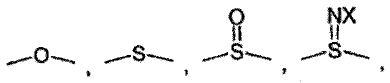 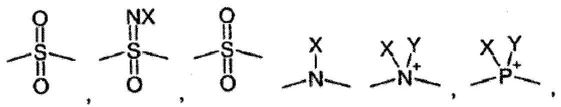 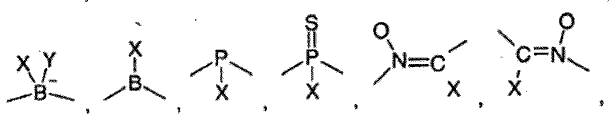 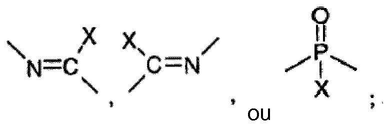 et
J est de la formule : 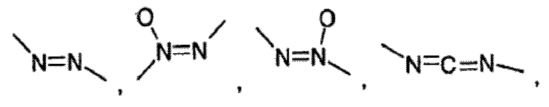 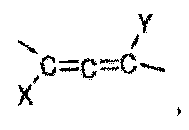 ou
-C≡C-
A) chaque X et Y est indépendamment un groupe de la formule :
-(L)ₘ-Z ;
et
B) m est un nombre entier allant de 0 à 8 ; et
C) pas plus de deux groupes E sont adjacents l'un à l'autre et aucun groupe J n'est adjacent à un autre ;
iii) chaque Z est indépendamment sélectionné parmi :
A) l'hydrogène, un halo, un cyano, un nitro, un nitroso, un azido, un chlorate, un bromate, un iodate, un isocyanato, un isocyanuro, un isothiocyanato, un pentafluorothio, ou
B) un groupe G, G étant un système de cycle non substitué ou substitué ; insaturé, partiellement saturé, ou saturé ; monocyclique, bicyclique, tricyclique, ou condensé ; carbocyclique ou hétérocyclique où ;
1) lorsque le système de cycle contient un cycle hétérocyclique à 3 ou 4 chaînons, le cycle hétérocyclique contient 1 hétéroatome ;
2) lorsque le système de cycle contient un cycle hétérocyclique à 5 chaînons, ou plus, ou un cycle hétérocyclique polycyclique, le cycle hétérocyclique ou hétérocyclique polycyclique contient de 1 à 4 hétéroatomes ;
3) chaque hétéroatome est indépendamment sélectionné parmi N, O, et S ;
4) le nombre de substituants va de 0 à 5 et chaque substituant est indépendamment sélectionné parmi X ;
b) W¹ et W² sont sélectionnés parmi F, Cl, Br, I, un alcoxy, un acyloxy, un alcoxycarbonyloxy, un aminocarbonyloxy, un alkylaminocarbonyloxy, un dialkylaminocarbonyloxy, un alkylsulfonyloxy, et un arylsulfonyloxy ;
c) à condition qu'au moins soit W¹, soit W² soit I ; et
d) le nombre total d'atomes différents de l'hydrogène est de 50 ou moins en tant qu'antagoniste de réponse à l'éthylène de plante par mise en contact de la plante avec ledit composé.

2. La méthode de la revendication 1 dans laquelle chaque W1 et W2 est I.

3. La méthode de la revendication 1 dans laquelle le composé est le 1,2-düodo-1-méthycyclopropane.

4. Une méthode pour inhiber la réponse à l'éthylène chez des plantes avec un composé cyclopropène comprenant les étapes consistant à :
A) fournir un composé de structure I, II, III ou IV 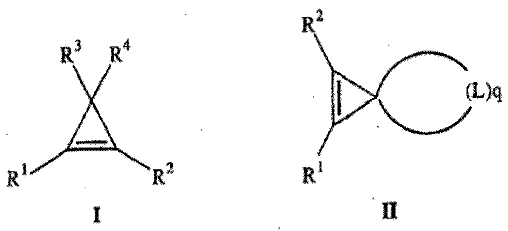 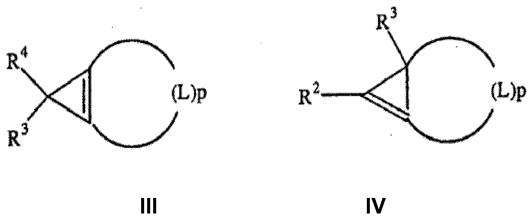 comprenant le fait de mettre en contact un composé de structure V, VI, VII ou VIII de la revendication 1, avec un agent réducteur ou nucléophile pour convertir le composé de structure V, VI, VII ou VIII en son composé analogue respectif de structure 1, Il, III ou IV, respectivement,
dans lesquelles :
a) chaque R¹, R², R³, et R⁴ est indépendamment un groupe de la formule :
-(L)ₙ-Z
i) p est un nombre entier allant de 3 à 10 ;
q est un nombre entier allant de 4 à 11 ;
n est un nombre entier allant de 0 à 12 ;
ii) chaque L est indépendamment sélectionné parmi un élément du groupe D, E, ou J ;
D est de la formule : 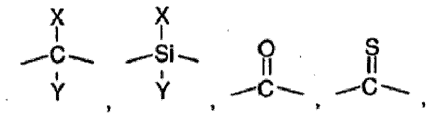 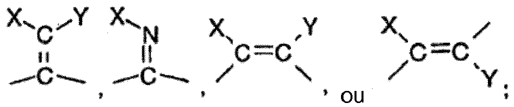 E est de la formule : 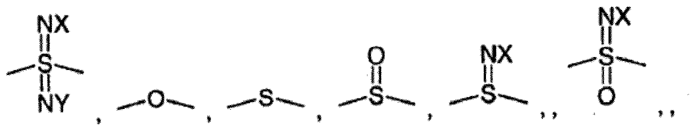 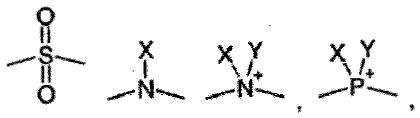 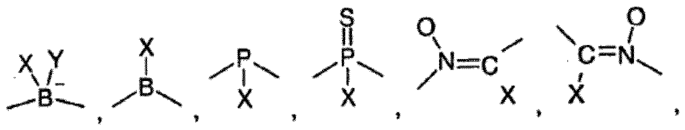 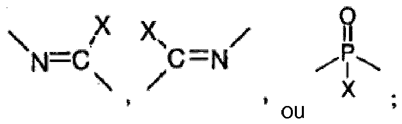 et
J est de la formule : 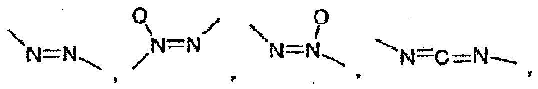 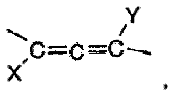
-C≡C-
ou
A) chaque X et Y est indépendamment un groupe de la formule :
-(L)ₘ-Z ;
et
B) m est un nombre entier allant de 0 à 8 ; et
C) pas plus de deux groupes E sont adjacents l'un à l'autre et aucun groupe J n'est adjacent à un autre ;
iii) chaque Z est indépendamment sélectionné parmi :
A) l'hydrogène, un halo, un cyano, un nitro, un nitroso, un azido, un chlorate, un bromate, un iodate, un isocyanato, un isocyanuro, un isothiocyanato, un pentafluorothio, ou
B) un groupe G, G étant un système de cycle non substitué ou substitué ; insaturé, partiellement saturé, ou saturé ; monocyclique, bicyclique, tricyclique, ou condensé ; carbocyclique ou hétérocyclique où ;
1) lorsque le système de cycle contient un cycle hétérocyclique à 3 ou 4 chaînons, le cycle hétérocyclique contient 1 hétéroatome ;
2) W¹ et W² sont sélectionnés parmi F, Cl, Br, I, un alcoxy, un acyloxy, un alcoxycarbonyloxy, un aminocarbonyloxy, un alkylaminocarbonyloxy, un dialkylaminocarbonyloxy, un alkylsulfonyloxy, et un arylsulfonyloxy ;
c) à condition qu'au moins soit W¹, soit W² soit Br ou I ; et
d) le nombre total d'atomes différents de l'hydrogène est de 50 ou moins, et B) mettre en contact la plante avec le composé.

5. La méthode de la revendication 4 dans laquelle l'agent réducteur est sélectionné dans le groupe consistant en des métaux, des réactifs organo-métalliques et des ions métalliques de faible valence.

6. La méthode de la revendication 4 dans laquelle l'agent nucléophile est sélectionné dans le groupe consistant en des mercaptans, des séléniures, des phosphines, des phosphites, Na2S, Na2Te, Na2S2O4, le sel de diéthylphosphite de sodium, KSCN, NaSeCN, la thio-urée, le diphényltellure et Nal.
